# EUROPÄISCHE PATENTSCHRIFT

(11) **EP 2 471 017 B1**
(45) Veröffentlichungstag und Bekanntmachung des Hinweises auf die Patenterteilung: **27.02.2019**
(21) Anmeldenummer: 10775696.7
(22) Anmeldetag: 24.08.2010
(51) Int. Cl.: G06F 19/00

(54) **VERFAHREN UND SYSTEM ZUR SPEICHERUNG UND AUSWERTUNG VON DATEN, INSBESONDERE VITALDATEN**
METHOD AND SYSTEM FOR STORING AND EVALUATING DATA, IN PARTICULAR VITAL DATA
PROCÉDÉ ET SYSTÈME DE MÉMORISATION ET D'ANALYSE DE DONNÉES, NOTAMMENT DE DONNÉES VITALES

(30) Priorität: 24.08.2009 DE 102009038391; 23.12.2009 DE 102009060553
(43) Veröffentlichungstag der Anmeldung: 04.07.2012
(73) Patentinhaber: vitaphone GmbH, 68165 Mannheim (DE)
(72) Erfinder: WALTER, Jörg, 33824 Werther (DE)
(74) Vertreter: Von Rohr Patentanwälte Partnerschaft mbB
(86) Internationale Anmeldenummer: PCT/EP2010/005162
(87) Internationale Veröffentlichungsnummer: WO 2011/023356

(56) Entgegenhaltungen:
- DE-A1-102007 016 447
- DE-A1-102008 003 321

## Beschreibung

Die vorliegende Erfindung betrifft ein Verfahren zur Speicherung und Auswertung von Daten gemäß dem Obergriff des Anspruchs 1, ein System zur Durchführung des erfindungsgemäßen Verfahrens gemäß Anspruch 10, ein Nutzergerät für das erfindungsgemäße System gemäß Anspruch 13 sowie eine Auswerteeinrichtung für das erfindungsgemäße System gemäß Anspruch 15.

Das erfindungsgemäße System ist zur Aufbereitung, Aggregation, Speicherung, Management und Weitergabe von Daten ausgebildet. Dieses System wird nachfolgend auch als intelligenter DataStore oder "iDataStore" bezeichnet. Es weist typischerweise ein sog. "embedded system" beim Nutzergerät auf, das Daten von einem oder mehreren Sensoren oder Sensorsystemen bekommt, für die es Datawarehousefunktionalitäten implementiert. Es ist typischerweise informatisch und räumlich sensornah platziert (oder im Sensorsystem integriert) und korrespondiert mit einer Auswertungseinrichtung, die die Daten episodisch (von Zeit zu Zeit) als Ganzes, aber meist in Teilen abruft, um sowohl systematische, als auch schnelle ad-hoc Analysen (Visualisierungen und interaktive Navigation) und andere Datenverarbeitungsschritte (Datamining) zu ermöglichen.

Die Verfügbarkeit von günstiger Rechenleistung, Speicherkapazität und preiswerten Sensoren ermöglicht zahlreiche, immer neue Anwendungen, die die Verarbeitung und Analyse von langen Signalaufzeichnungen beinhalten. Tragbare, batteriebetriebene Systeme oder Nutzgeräte können Sensorsignale aufzeichnen, analysieren und ggf. autonom über eine Funkverbindung an eine Empfangseinheit oder Auswerteeinrichtung weiterleiten, die die Daten manuell oder automatisch weiterverarbeitet.

Die DE 10 2008 003 321 A1 (D1) betrifft einen mobilen EKG-Rekorder zur kardiologischen Überwachung von Patienten, bei dem ein abgenommenes EKG-Signal abschnittsweise als EKG-Abschnitt gespeichert, analysiert und übertragen werden kann, wobei eine automatische Speicherung von EKG-Signalen bzw. EKG-Abschnitten bei Detektion einer definierten Herzrhythmusstörung möglich ist.

Die DE 10 2007 016 447 A1 (D2) betrifft eine Vorrichtung zur Ermittlung von Biosignalen anhand derer ermittelt wird, wie hoch das individuelle Risiko der untersuchten Person ist. Eine Signalauswertung über eine vorgebbare Zeitspanne wird dadurch unterstützt, dass der Sensor mit einer ersten Speichereinheit zur Abspeicherung eines erfassten Messsignals verbunden ist.

Aufgabe der vorliegenden Erfindung ist es, eine genauere und/oder resourcenschonendere Auswertung von Sensordaten zu ermöglichen.

Diese Aufgabe wird gelöst durch ein Verfahren gemäß Patentanspruch 1, ein System gemäß Patentanspruch 10, ein Nutzergerät gemäß Patentanspruch 13 und eine Auswerteeinrichtung gemäß Patentanspruch 15. Vorteilhafte Weiterbildungen sind Gegenstand der Unteransprüche.

Der Erfindung liegt der Gedanke zugrunde, "nur die interessanten" Daten behandeln und/oder übertragen zu wollen, wobei im Zuge der Auswertung die genaue Definition von "Interessantheit" erst entsteht bzw. sich komplett verschieben kann und damit kaum vorhersehbare Datenausschnitte benötigt werden.

Zum Beispiel sind im Bereich der Telemedizin Vitaldaten, wie das EKG-Signal, für das kardiale Monitoring und die Diagnostik von hoher Bedeutung. Holster-EKGs werden 24 Stunden getragen und der ganze Datensatz archiviert. Event- und Loop-Recorder werden länger getragen, um Arrhythmien zu detektieren und übertragen Kurzzeit-EKG in digitaler Form.

Eine Auswertung eines Holster-EKGs oder sonstiger längerer Datensätze ist tendenziell aufwendig. Es ist üblich, den gesamten Datensatz auf ein Auswertesystem zu übertragen, zu sichten und auszuwerten. Weiterhin ist die Laufzeit und/oder Auflösung dieser Systeme begrenzt. Event- und Loop-Rekorder können länger getragen werden, insbesondere vor dem Hintergrund, daß nur aufgrund einer zuvor definierten Interessantheitsmetrik, insbesondere bestimmter Schwellwerte oder Zustandswechsel, eine Aufzeichnung aktiviert wird (z. B. Brachykardieerkennung mit einer Herzrate größer 160 bpm). Dieses Verfahren verhindert jedoch, daß relevante Zeitbereiche vor Eintritt eines auslösenden Ereignisses ausgewertet werden können.Nachträgliche Korrelationsanalysen, Trendanalysen, Analysen von EKG-Signalen oder von sonstigen Daten können insbesondere erhellend sein, wenn weitere Informationen verfügbar werden, wie zum Beispiel Befindlichkeitsveränderungen, Medikationsänderungen und andere auffällige Signalaufzeichnungen. Hierfür bedarf es einer Signalspeicherung und Übertragung zur Analyseeinheit der Auswerteeinrichtung. So ist es notwendig, daß für solche Verfahren geeignete Datensätze zur Verfügung stehen, was bei Event- und Loop-Rekordern in der Regel nicht der Fall ist. Zudem muß für solche Verfahren bisher der gesamte Datensatz mit einem entsprechend großen Datenvolumen übertragen werden. Dies ist insbesondere abhängig von der verfügbaren Datenübertragungsrate oder Bandbreite, zeitaufwendig und kostenintensiv.

Heute sind Digitalspeicher prominent. Die Übertragung geschieht auf drei Wegen: (i) Medienaustausch (Speicherchip, früher Kassette), (ii) kabelgebunden (z.B. USB-Verbindung) oder (iii) kabellos.

Die Vorteile der kabellosen Funkverbindung sind die bessere Gerätekapselung (Dichtigkeits-, Reinigbarkeits- und elektrische Isolationsanforderungen) und die höhere Bequemlichkeit (Gerät kann vor Ort bleiben, kein Kabelsalat, keine Speicherkartenlogistik). Die Nachteile der Funkverbindung bestehen beispielsweise in dem Risiko, daß die Datenübertragung nicht immer zuverlässig verfügbar ist (Störung, Kanalbelegung, Netzdeckung), im erheblichen Energieverbrauch (je nach Reichweite), in der auftretenden Bandbreitenlimitierung (damit der praktische Zeitbedarf) und ggf. der zusätzlichen Datenübertragungsgebühren (z.B. GSM/UMTS). Der Energieverbrauch limitiert die Batterielebensdauer und Batterie-Iade- oder Batterie-Austausch-Zyklen und beeinflusst die Gebrauchstauglichkeit des Systems.

In einem konventionellen Sensordaten-Messsystem ergeben sich folgende praktische Probleme:
- Es besteht ein (latentes) Interesse an der Datenverfügbarkeit in hoher Signalauflösung, beispielsweise zum Vergleich der jüngsten Signalaufzeichnungen (ggf. real-time) mit bestimmten historischen Daten. Tatsächlich wird mitunter nur ein sehr geringer Teil der Signalbeobachtung ausgewertet - welcher ist aber vorher unklar.
- Es fallen mit der Zeit mitunter sehr große Datenmengen an, die übertragen und gespeichert werden müssen, um für die Auswertung auf einem Auswertungssystem (Rechnersystem, ggf. verteilt) zur Verfügung zu stehen.
- Auswertungen sind natürlich erst möglich, wenn die Daten zuvor übertragen worden sind. Damit sind schnelle ad-hoc Auswertungen nur möglich, wenn präventiv sämtliche relevanten Daten periodisch oder dauernd übertragen werden, was zu den oben genannten Übertragungskosten für die große Datenmenge führt - egal ob sie genutzt wird oder nicht.
- Die Datenhaltungskosten (etwa in einem Datenzentrum, insbesondere bei Archivierungs- und/oder Weiterverarbeitungs- und/oder Begutachtungspflicht) und die Übertragungskosten können schnell zur Last werden und den Nutzer zur Abschaltung bzw. grundsätzlichen Nichtnutzung der "teuren" Datenübertragung motivieren.

Die vorliegende Erfindung unter dem zusammenfassenden Kennwort "iDataStore" vermeidet diese unnötigen und möglicherweise prohibitiven Übertragungskosten sowie Wartezeiten und bietet damit große Vorteile in resourcenlimiterten Anwendungen.

Das iDataStore-System speichert Daten, typischerweise digitalisierte Sensormesssignale, (informatisch) nahe an der Datenquelle (z.B. auf nichtflüchtigen Flash-Speicher). Die analogen und digitalen Komponenten der Sensorsignalvorverarbeitung können mit dem iDataStore auch in einem Gesamtsystem kombiniert werden. Alternativ können die Signale statt von einem oder mehreren Sensoren auch teilweise via Kabel oder kabellos von einem anderen digitaldatenliefernden System (z.B: GPS-Modul) übernommen werden.

Vorzugsweise findet diese Datenspeicherung in einem batteriebetriebenen System oder Nutzergerät statt, das vorzugsweise netzunabhängig oder mobil betreibbar ist. Das Nutzungsgerät ist also insbesondere transportabel.

Die Daten werden zudem, insbesondere im Nutzergerät, zeitnah aggregiert und insbesondere in zeitlich hierarchischer Form, d. h. für eine hierarchische Datennavigation so präpariert, dass sie von der Auswerteeinheit über eine digitale Abfrageschnittstelle in diversen zeitlich und/oder semantisch komprimierten Formen ausgelesen werden können.

Das Gesamtsystem erlaubt die interaktive Navigation über die Auswerteeinrichtung in großen Datenmengen auf dem sensornahen iDataStore-System.

Es unterstützt die sog. Drill-down Möglichkeit in hierarchisch aggregierten Daten. Werden z.B. durch visuelle Inspektion bestimmte Datenbereiche als interessant befunden, wird die nächst höhere Auflösungsstufe (durch eine Abfrage) ausgelesen und dargestellt. Es sind also Daten im Nutzungsgerät besonders bevorzugt in einer ersten und zusätzlich mindest in einer zweiten, vorzugsweise auch einer dritten, Aggregationsstufe abgespeichert und/oder verfügbar. Eine Analyse von abgeleiteten Attributen ist vorzugsweise kombinierbar.

Eine Aggregation von aufgezeichneten Daten kann auf vielfältige Art und Weise vorgenommen werden. Insbesondere werden aufgezeichnete Rohdaten durch ein Verfahren in geeigneter Weise abstrahiert. Vorzugsweise ergibt die Abstraktion einen repräsentativen Überblick über die Rohdaten, ohne daß diese jedoch verworfen werden. Somit ergibt sich neben den Rohdaten ein erster Datensatz aggregierter Daten.

Mit demselben oder einem anderen Verfahren ist vorgesehen, die bereits aggregierten Daten ein weiteres Mal zu aggregieren. Auf diese Weise werden unterschiedliche Aggregationsebenen erhalten, die mit zunehmender Aggregationsstufe eine verminderte Datenmenge aufweisen und hierdurch einen Überblick ermöglichen.

In Bezug auf die Übertragung von Daten von einem Nutzergerät zu einer Auswerteeinheit bedeutet dies, daß nicht zwingend der gesamte Datensatz übermittelt werden muß. Im Gegensatz ermöglicht die beschriebene Aggregation einen hierarchischen Zugriff auf die Daten.

Beispielsweise werden bei einer ersten Anforderung von Daten nicht die gesamten Rohdaten oder lediglich ein Ausschnitt dieser Rohdaten übermittelt, sondern es werden aggregierte Daten, insbesondere Daten der höchsten oder einer höheren Aggregationsebene oder mit dem geringsten oder einem geringerem Datenvolumen, übertragen. In einem nächsten Schritt ist es möglich, detailliertere Daten anzufordern, vorzugsweise eine niedrigere oder einen Ausschnitt einer niedrigeren Aggregationsebene. So ist eine detailliertere Auswertung oder Analyse bestimmter oder besonders interessanter Datenbereiche möglich, ohne den gesamten Datensatz übermitteln zu müssen. Das geschilderte Verfahren läßt sich bis hin zu den Rohdaten fortsetzen und entspricht dem erwähnten Drill-Down-Verfahren zum Zugriff auf die hierarchisch aggregierten Daten.

In Bezug auf die Aggregation der Daten ist vorgesehen, unterschiedliche Verfahren anzuwenden oder miteinander zu kombinieren. So ist erfindungsgemäß vorgesehen, auf einen Satz Rohdaten unterschiedliche Aggregationsverfahren alternativ oder kumulativ anzuwenden. Diese wiederum können ebenfalls unterschiedliche Aggregationsebenen aufweisen.

Das Datenvolumen sinkt mit zunehmender Aggregation. Daher ist es möglich, sich die mehrstufig aggregierten Daten als Pyramide vorzustellen, wobei vorzugsweise die Rohdaten mit dem größten Datenvolumen das Fundament der Pyramide darstellen und hierauf aufbauend mit jeder Aggregationsstufe oder -ebene ein verringertes Datenvolumen einhergeht. Übertragen auf mehrere Aggregationsverfahren bedeutet dies, daß basierend auf den selben Rohdaten mehrere solche Aggregations-Pyramiden existieren können.

Es ist weiterhin möglich, daß für unterschiedliche Verfahren auch eine unterschiedliche Anzahl von Aggregationsstufen existieren können. Zudem ist es möglich, auch innerhalb einer Aggregations-Pyramide oder Aggregations-Hierarchie unterschiedliche Aggregations-Verfahren zu kombinieren.

Je nach Anwendung und Signal können spezifische zeitliche/semantische Aggregationen verwendet werden:
- Eine Möglichkeit der Aggregation ist zeitliches subsampling und/oder digitales Filtern. Subsampling oder Unterabtasten bezeichnet in Bezug auf ein einfaches zeitliches Signal in der Regel Verfahren, bei denen das Signal mit weniger als der doppelten Bandbreite der höchsten auftretenden Frequenz abgetastet wird. Ein solches Unterabtasten kann sowohl während eines Digitalisierungsvorgangs als auch nachträglich auf Basis eines bereits digitalisierten Signals durchgeführt werden. Liegen Frequenzen des Signals oberhalb der doppelten Abtastfrequenz, ist das Ergebnis nicht mehr eindeutig, insbesondere da Frequenzen oberhalb der doppelten Abtastfrequenz im digitalen Signal als Differenzfrequenz zur halben Abtastfrequenz erscheinen. In Bezug auf die Aggregation ist es durch das Unterabtasten möglich, trotz der fehlenden Eindeutigkeit, zum Beispiel über Musteranalysen, das Signal weiter auszuwerten. In sofern weisen Verfahren zur Unterabtastung Analogien zu Kompressionsverfahren auf, bzw. Kompressionsverfahren können Unterabtastungen aufweisen.
- Eine weitere Klasse von Aggregationsverfahren verwenden Kompression der Daten (u.a. verlustbehaftet) angepasst an das (Sensor-) Signal, um möglichst relevante Signalmerkmale zu erhalten. Beispielsweise ist es möglich, mit Methoden basierend auf einer Wavelet-Transformation, diskreter Kosinustrasformation (DCT) oder dergleichen einen insbesondere einstellbaren Anteil von Informationen pro Zeitintervall zu erhalten bzw. zu verwerfen. Hierbei ist es bevorzugt, Verfahren wie Unterabtastung, statistische Analyse, Berechnung abgeleiteter Signalbeschreibungen, nichtlineare Transformationen, insbesondere FFT, DCT, Wavelet-Transformation, jeweils insbesondere mit komprimierender Ausdünnung, und/oder Verfahren des maschinellen Lernens zu verwenden. Neben Extremwert-Methoden bieten sich diverse weitere Kompressionsverfahren zur Aggregation von Daten an, die jedoch dem Fachmann bekannt sind und keiner näheren Erläuterung bedürfen.

Alternativ oder zusätzlich ist es möglich, Peak-Level oder Maximalwertbasierte Kompressionsverfahren zu nutzen, um beispielsweise Maxima oder Minima eines Signalverlaufs, insbesondere innerhalb eines bestimmten Zeitintervalls zu bestimmen. In Abhängigkeit von der Größe der Zeitintervalle und/oder der Genauigkeit der Darstellung des Extremwerts kann auf einfache und wirksame Weise über dieses Kompressionsverfahren eine hierarchische Aggregation erzielt werden.
- Statistische Beschreibungen (Mittelwerte, Standardabweichung, höhere Momente, Histogramme, ggf. auch Periodogramme, Poincaréplots) sind weitere bevorzugte Verfahren zur Aggregation von Signalen. Auch statistische Verfahren führen durch die Datenreduktion zu einer Kompression, die insbesondere ebenfalls verlustbehaftet sein kann. Statistische Beschreibungen oder statistische Methoden können jedoch in vorteilhafter Weise dazu verwendet werden, Datenbereiche mit Abweichung zu lokalisieren, die eine Auswertung vereinfachen oder ermöglichen können.
- Klassifikation des Signals in Kategorien zur Erlangung einer semantischen Beschreibung und damit Einschätzung der Vorgänge im Zeitintervall. Hierfür können bevorzugt Techniken und Verfahren des maschinellen Lernens (vgl. US 2010 191 685 A1) verwendet werden.
- Zudem kann die Messgüte und der Messsystemzustand und -kontext beurteilt und notiert werden (z.B. elektrische Kontaktbeurteilung).
- Diese Schritte können rechen- und speichereffizient zeitnah am Ende von vorgegebenen Zeitintervallen (z.B. Minute, Stunde, Tag etc.) durchgeführt werden, vorzugsweise wodurch später eine performante Abfrage - insbesondere auch ohne Übertragung der Rohdaten - zu ermöglichen.

Die (Roh-)Daten in hoher zeitlicher Auflösung werden bei der Erfindung vorzugsweise nicht verworfen, sondern so lange wie möglich und gewünscht verfügbar gehalten. Wird eine definierte Speicherkapazitätsobergrenze erreicht, werden alte speicherintensive Signaldaten (auf Wunsch mit Ausnahme von Stichproben und höheren Aggregationsstufen) sukzessive gelöscht und durch neuere Daten ersetzt.

Die Aggregation bzw. die Auswertung von Daten wird zumindest im Wesentlichen bereits im (transportablen und/oder netzunabhängigen) Nutzergerät ausgeführt. In vorteilhafter Weise ist es so möglich, im Zuge der Auswertung auf bereits vorverarbeitete Daten zurückzugreifen. Hierdurch kann ein signifikanter Zeitvorteil und/oder ein Datenübertragungsvorteil erzielt werden. Beschränkt sich das Interesse auf eine Überblicksinformation, brauchen keine Details, die in den "Rohdaten" (bzw. hochauflösenden Daten) stecken, übertragen werden. Das spart nicht nur den Übertragungsaufwand (Zeit, ggf. Energie, Leitungskosten) sondern schützt bei Vitaldaten einer Person zusätzlich dessen Privatsphäre. Zeitlich hochaufgelöste Daten enthalten potentiell Informationen, die zum Verbesserung von persönlichen System- und Verhaltensprofile genutzt werden kann. Ist eine Nutzung zweckdienlich und vereinbart, kann die nachträgliche Verfügbarkeit von Detaildaten von großem Wert sein, beispielsweise für das Training von spezialisierten Verhaltensänderungsdektoren (z.B. Notfalldetektoren) oder der retrospektiven Analyse von schleichenden Veränderungsprozessen. Der iDataStore kann Daten, insbesondere auch hochauflösende oder Rohdaten, verfügbar halten und kann ein latentes Interesse nachträglich befriedigen. Für den Schutz von missbräuchlichen können zudem Zugriff geeignete Schutzmassnahmen getroffen werden. Für eine überblicksartige Auswertung, bietet das vorgeschlagene Verfahren folglich mehrfache große Vorteile. Ein weiterer Vorteil ergibt sich durch den beschriebenen, insbesondere interaktiven und/oder explorativen Drill-Dow-Prozess für einen vorzugsweise stufenweisen Zugriff auf Daten unterschiedlicher Aggregationsstufen, wie eingangs erläutert.

Die Anzahl der im Nutzergerät zu berechnenden Aggregationsebenen, die Anzahl von Aggregationsverfahren, die insbesondere miteinander kombiniert oder parallel eingesetzt werden, und die Auswahl der Verfahren zur Aggregation können erfindungsgemäß durch voreingestellte Prioritäten, durch verfügbare Rechenleistung, durch einen internen Status und/oder davon abhängig sein, in welchem Ladezustand sich das Nutzergerät befindet und/oder wie das Leitungsprofil konfiguriert wurde. Der Ladezustand ist insbesondere bei einem netzunabhängigen Nutzergerät, also einem Nutzgerät, das vom Stromnetz und/oder von einer drahtgebunden bzw. kontinuierlichen drahtgebundenen Verbindung unabhängig ist, relevant.

Eine Priorisierung von verschiedenen Aggregationsverfahren, deren Kombination und Aggregationstiefe kann beispielsweise derart beeinflußt werden, daß bei einem mobilen Betrieb oder bei einem geringen Ladezustand wenige oder wenig rechenintensive Aggregationsverfahren verwendet werden oder die Anzahl von Aggregationsebenen begrenzt bzw. die Aggregationstiefe reduziert wird. So ist es erfindungsgemäß möglich, die Aggregationsverfahren aus einer Mehrzahl von Aggregationsverfahren auszuwählen und/oder die Aggregationstiefe für den Fall eines guten Ladezustands oder bei Netzbetrieb entsprechend auszuweiten und insbesondere in diesem Fall auch komplexe und/oder rechenaufwendige Verfahren anzuwenden.

Vorzugsweise weist das Nutzergerät einen Energiespeicher, wie einen Akku oder eine Batterie, auf. Hierdurch kann in vorteilhafter Weise ein netzunabhängiger oder mobiler Betrieb des Nutzergerätes ermöglicht werden. Weiter weist das Nutzergerät vorzugsweise eine Vorrichtung zur Bestimmung des Ladezustandes dieses Energiespeichers auf. Erfindungsgemäß ist es möglich, daß die Recheneinheit des Nutzergerätes Informationen über den Ladezustand von dieser Vorrichtung erhält und abhängig von dieser Information, insbesondere abhängig von einem Entscheidungskriterium, wie einem Schwellwert, die beschriebene vom Ladezustand abhängige Auswahl von Aggregationsverfahren aus einer Mehrzahl von Aggregationsverfahren durchführt.

Vorteilhaft ist eine Aggregation, Vorverarbeitung, Analyse der Daten bereits im Nutzergerät auch vor dem Hintergrund, daß während der Aufzeichnung von Daten relativ viel Zeit zur Verfügung steht. Bei einer Auswertung jedoch die notwendigen Informationen möglichst unmittelbar zur Verfügung stehen sollten. Daher kann eine Vorverarbeitung im Nutzergerät insbesondere deshalb stromsparend realisiert werden, wenn entsprechende Berechnungen langsam und/oder mit geringen Taktraten erfolgen.

Der iDataStore wird typischerweise von einem korrespondierenden Verarbeitungssystem/Auswerteeinrichtung zwecks (ad-hoc-)Darstellung und Analyse abgefragt.

Bei Erreichen von besonderen Zuständen oder Eintreffen von bestimmten Ereignissen (z. B. kritische Vitalzustände) kann der iDataStore alternativ oder zusätzlich auch aktiv eine Nachricht an die Auswerteeinrichtung oder eine zuständige Zentralstelle (z.B. telemedizinisches Servicezentrum) abschicken. Zur Sicherstellung der Systemverfügbarkeit kann eine regelmäßige "alive"-Meldung (ähnlich einem "Totmannschalter"), insbesondere in Verbindung mit einer Batteriezustandsmeldung vereinbart werden, bzw. vorgesehen sein.

Bei herkömmlichen Event-Rekordern ist es möglich, eine Reaktion auf ein bestimmtes Ereignis vorzudefinieren. Im erfindungsgemäßen System können in vorteilhafter Weise auch die aggregierten Daten verwendet werden, um auf diesen basierend entsprechende Events auszulösen. Beispielsweise werden durch das Nutzergerät Sensordaten erfaßt und durch die Rechnereinheit abgespeichert. In bestimmten Zeitabständen kann ein Aggregationsverfahren verwendet werden, um die aufgezeichneten Daten des vergangenen Intervalls zu aggregieren. Aggregationsverfahren können Analyseverfahren sein, solche aufweisen oder eine Analyse ermöglichen. Folglich kann die Aggregation von Rohdaten dazu führen, daß ein bestimmtes Ereignis oder ein Zustandswechsel durch Anwendung eines Aggregationsverfahrens automatisch identifiziert werden kann. Vorzugsweise ist es möglich, diese Information zu verwenden, um eine Aktion im Nutzergerät auszulösen.

Die Rechnereinheit des Nutzergerätes kann beispielsweise im Zuge oder abwechselnd mit einer Anwendung eines Aggregationsverfahrens bereits aggregierte Daten mit einer zuvor definierten Interessantheitsmatrix oder einem Bewertungskriterium vergleichen. Wird ein solches Kriterium oder eine solche Interessantheitsmatrix erfüllt, kann eine vordefinierte Reaktion des Nutzergerätes ausgelöst werden. Beispielsweise kann das Nutzergerät in diesem Fall die Teilmenge der Daten markieren, in der ein Kriterium oder eine Interessantheitsmatrix erfüllt ist. Weiter kann ein Zustandswechsel, insbesondere durch die Auswerteeinrichtung abrufbar abgespeichert oder aktiv signalisiert werden. Eine Signalisierung kann beispielsweise ein Warnsignal wie ein Licht- oder Tonsignal sein. Das Nutzergerät kann jedoch auch, insbesondere über eine Funkverbindung wie eine Mobilfunkverbindung, eine zuständige Zentralstelle wie ein telemedizinisches Servicezentrum kontaktieren, insbesondere um dort eine Warnung bezüglich des detektierten Zustandswechsels auszulösen.

Es ist möglich, daß die Zentralstelle oder auch das Nutzergerät aufgrund eines Zustandswechsels eine Auswerteeinrichtung kontaktiert und/oder einer Auswerteeinrichtung entsprechende Informationen wie eine Warnung übermittelt.

Datenmuster als Kriterium zur Erkennung einer Zustandsänderung werden in diesem Zusammenhang auch abgeleitete Features, digitale Fingerabdrücke oder Fingerprints genannt. In vorteilhafter Weise ist es mit dem erfindungsgemäßen System möglich, solche Fingerprints nicht nur vorab zu definieren, sondern auch im Nachhinein festzulegen. So ist es möglich, einem Nutzergerät nach der Aufzeichnung einen entsprechenden digitalen Fingerabdruck zu übermitteln und die Analyse basierend auf dem digitalen Fingerabdruck auf dem Nutzergerät, insbesondere mit den aggregierten Daten, durchzuführen. Eine Antwort kann somit spezifisch und/oder aggregiert erfolgen. Ein solches Verfahren ist daher besonders vorteilhaft bei Datenverbindungen mit einer beschränkten Bandbreite, insbesondere Funkverbindungen oder Mobilfunk-Verbindungen. Ein digitaler Fingerabdruck kann erfindungsgemäß vorzugsweise eine Bedingung, mehrere Bedingungen und/oder eine Kombination von Bedingungen sein. Diese Bedingungen können sich auch auf Daten mehrerer unabhängiger Sensoren gleichzeitig beziehen.

In einer bevorzugten Ausführungsform ist das erfindungsgemäße Verfahren interaktiv, läßt also vorzugsweise anfragen und, insbesondere dynamische, Reaktionen auf diese Anfragen zu.

Wird durch eine, insbesondere vordefinierte, Interessantheitsmetrik, wie beispielsweise ein Schwellwert oder ein digitaler Fingerabdruck, ein Zustandswechsel erkannt, kann das System auf unterschiedliche Weise reagieren. Es ist möglich, den Zustandswechsel für eine spätere Auswertung zu verzeichnen, oder auch aktiv auf diesen zu reagieren oder aufmerksam zu machen, insbesondere durch eine akustische oder optische Warnung oder durch Versenden einer Nachricht. Letzteres kann vorzugsweise drahtlos, insbesondere über das Mobilfunknetz, geschehen.

Das Auswertesystem kann via Kabel oder Funk mit für eine gesicherte Kommunikation geeigneten Mechanismen (inkl. geeigneter Autorisierung und Authentifikationsverfahren) mit dem iDataStore kommunizieren (insbesondere via XML-Nachrichten).

Eine Datenübertragung mittels Medienwechsel, insbesondere durch den Austausch von Speicherkarten, ist verhältnismäßig aufwendig und verschleißbehaftet. Daher ist eine Datenübertragung mittels Medienwechsel vorzugsweise nur dann vorgesehen, wenn eine Sicherheitskopie erstellt werden soll, der vorhandene Speicher nicht ausreicht und/oder ein möglicher Defekt vorliegt.

Für ad-hoc Abfragen ist, insbesondere als Auswerteeinheit, z.B. ein, vorzugsweise über Bluetooth, Zigbee oder andere kabellose Kommunikationsverfahren verbundenes, mobiles Gerät wie ein PDA, Smartphone oder Notebook, vorzugsweise mit spezieller Navigations- und Analysesoftware, nutzbar und unterstützt vorzugsweise schrittweise Analysemethoden (z.B. visuelles Datamining).

Zur Abfrage der aggregierten Daten vom Nutzergerät können für das jeweilige Übertragungsverfahren standardisierte Protokoll-Gerüste kombiniert werden mit systemspezifischen Anfragen, die insbesondere eine hierarchische Navigation durch die Daten, insbesondere mit der beschriebenen Drill-Down-Verfahrensweise ermöglicht.

Das Abfrageprotokoll umfasst vorzugsweise einige generische Anfragen nach:
- Liste der Datentypen und verfügbaren Datenmengen und/oder
- einem real-time Datenstrom (mit minimaler Latenzzeit und bestimmbarer Auflösung) und/oder
- Sequenz von Rohsignal oder abgeleiteten Daten innerhalb eines Intervalls (nach lokaler Zeit oder Ids). Die Antwort ist datentypspezifisch.
- Häufigkeiten von abgeleiteten Daten innerhalb von Abfrageintervalle (z.B. klassifizierte Arrythmietypen pro Minute, Stunde, Tag, etc.)

Das Kopieren von Datensätzen in einen und Verwendung von einem sog. "caching proxy" werden vorzugsweise unterstützt, ebenso die Abfrage von Systemzuständen und Kommandierung von Löschprozess- und Aggregationsparametern.

Sofern die Rechenkapazität des iDataStores dies zulässt, kann die Abfrage neben einer XML-basierten oder sonstigen Formulierungen auch in einer SQL-artigen Anfrage unterstützt werden. Damit sind komplexere Restriktionen und Selektionen des Antwortdatensatzes besonders effektiv formulierbar, um interessante Datenbereiche schnell zu identifizieren (siehe z.B. sqlite.org). Erfindungsgemäß ist es jedoch auch möglich, sonstige Daten mit Architekturen und/oder Zugriffsverfahren auf diese Datenbankstrukturen anzuwenden.

Die Erfindung ist für eine Vielzahl von Signaldaten einsetzbar. Bevorzugt ist der Einsatz im häuslichen Umfeld und im Vitaldatenbereich. So können bei EKG-Signalen beispielsweise folgende Abfragen für sich oder in belieber Kombination durchgeführt werden:
- near-real-time EKG-Signal
- Rohsignal von Zeitstempel von ... bis...
- Signal in Auflösung ... von ... bis...
- Tachogramm = Herzrate (HR) von ... bis... pro... (Minute, Stunde,..)
- HR,
- HR-Standardabweichung,
- HR-Histogramm (% der Zeit HR zwischen 60..70 bps, / 70..80 bps, ...)
- Anzahl Schläge in Schlagklasse (Normal, Extrasystolen, ...)

### Beispiele für Sensoren sind:

Aktivitätsensoren, Beschleunigungssensoren, Pedometer, Smart-Metering-Sensoren, Verbrauchssensoren für ADL (activity of daily life), Präsenzsensoren, PIR (Passive Infrarot) Sensoren, Drucksensoren insbesondere für Boden, Bett und/oder Sitzgelegenheit, Sensoren für Feuchtigkeit, Stress und andere psychische Zustände (Hautimpedanz), Dehnungssensoren, akustische Sensoren (Atemgeräusch, Umgebungsgeräusch etc.) sowie Licht-, Bewegungs-, Distanz-, Magnet-, Kontakt-, Schließzustands-, Rauch- und/oder Gas-Sensoren.

Die vorgenannten Sensoren können für sich oder in verschiedener Kombination miteinander verwendet werden, wobei Daten mehrerer Sensoren dazu verwendet werden können, weitere Daten und/oder Informationen aus den Messdaten zu gewinnen bzw. generieren.

### Beispiele für zu messende Daten sind:

Vitaldaten, EKG Signale (s.o.), auch in Kombination mit einer Weste und Defibrilatoren, EEG, Temperatur, Druck (Plethysmograph (z.B. Brustvolumenschätzung)), Verbrauchsdaten (Energie- und Mengenflüsse: Strom, Gas, Wasser), Bewegungsdaten, (3D bis 6D-) Beschleunigungsdaten, insbesondere bestimmt per Accelerometer und Gyrometer, Umgebungsdaten (Luftdruck, insbesondere bestimmt durch ein Höhenbarometer, Ort, Lage), Lokalisierungsdaten (GPS, Funkzellen-IDs).

Beispiele für abgeleitete Daten sind (u.a.) Schätzungen über Zustände, Vorgänge oder Aktivitäten, ADL (Activity of Daily Life) und Beteiligte.

Anhand eines Elektrokardiogramms (EKG) sollen im Folgenden einige Details der vorliegenden Erfindung genauer erläutert werden.

Ein bevorzugtes Ausführungsbeispiel weist ein Nutzergerät und eine Auswerteeinrichtung auf. Das Nutzergerät wird vorzugsweise in der Nähe eines Patienten angeordnet und ist netzunabhängig betreibbar. Das Nutzergerät kann ein Gehäuse aufweisen, das in einer Hosentasche, Jackentasche oder Hemdtasche verstaut werden kann. Weiter ist es besonders bevorzugt, das Nutzergerät in einen Gurt oder Gürtel zu integrieren, da dies in besonders einfacher und gleichwohl effektiver Weise ermöglicht, Sensoren an das Nutzergerät anzuschließen. Andere Ausführungsformen unterstützen die einfache Installation in Räumen.

Das Nutzergerät kann unterschiedliche Sensoren aufweisen oder mit diesen verbunden werden. Insbesondere ist eine Verbindung mit den oben genannten Sensoren möglich. Das Nutzergerät weist weiter eine Recheneinheit auf, die Sensordaten erfassen, verarbeiten und/oder speichern kann. Hierzu weist die Rechnereinheit vorzugsweise einen Speicherbereich auf oder der Rechnereinheit ist ein Speicher zugeordnet. Dieser Speicherbereich wird besonders bevorzugt durch Flash-Speicher realisiert, kann jedoch auch eine Festplatte oder sonstige Speicher aufweisen.

Das Nutzergerät des erfindungsgemäßen Systems, insbesondere eines EKG-Systems weist weiter eine Schnittstelle zur Auswerteeinrichtung auf. Diese kann insbesondere eine Funkverbindung wie eine Mobilfunkverbindung oder eine drahtgebundene Schnittstelle aufweisen, In vorteilhafter Weise ist eine Funkverbindung möglich, um auch mobil auf Sensordaten zuzugreifen.

Die Auswerteeinrichtung weist vorzugsweise eine Anzeigeeinheit, wie ein Display auf. Auf diesem Display können vorzugsweise durch das Nutzergerät oder von dem Nutzergerät übermittelte Daten dargestellt werden.

Für eine Aufzeichnung, insbesondere ein EKG kann eine Interessantheitsmetrik in Trigger, deren Ableitungen, eine Kombination dieser und eine Uninteressantheitsmasse unterteilt werden. Zu den bekannten Triggern gehören Werte für die Tachycardia, die Bradycardia, das Vorhofflimmern, das Kammerflimmern sowie die (Gesamt-)Aktivität und die Pause der Aktivität des Patienten.

Aus diesen Triggern können Ableitungen gebildet werden. Insbesondere ist es möglich, Aktivitätintegrale zu bilden. Rückschlüsse auf die Herzratenvariabilität und die Herzratenturbulenzen sind möglich. Die Aktivität eines Patienten kann beispielsweise eingeteilt werden in liegende und sitzende Lage oder die tägliche Aktivität. Weiter ist es zusätzlich möglich, Lokalisierungsinformationen wie GPS-Informationen oder Signale von Trägheitssensoren zu verwenden. Zudem ist eine beliebige Kombination der vorgestellten Trigger und ihrer Ableitungen möglich. Ein Fingerpriniting oder digitaler Fingerabdruck kann eine Kombination dieser darstellen.

Neben den aufgezählten Bespielen für die Interessantheitsmetrik gibt es auch Kriterien, die gegen eine Zustandsänderung sprechen und auf eine Uninteressantheit hinweisen. So gibt es bekannte Konstellationen von Sensorsignalen, die auf eine Fehlmessung hindeuten. Hohe Aktivitäten der Brustmuskulatur, als auch mechanische Belastungen können zu Störsignalen führen. Beispielsweise können die Bewegungen beim Zähnputzen dazu führen, daß das abgeleitete EKG-Signal dem bei einem Vorhofflimmern ähnelt. Durch zusätzliche Sensorik kann anhand der Aktivität des Patienten jedoch eine entsprechende mögliche Fehlmessung detektiert werden.

Ein erfindungsgemäßes System ist vorzugsweise derart ausgestaltet, daß es auf einen oder mehrere Trigger oder auf eine oder mehrere ihrer Ableitungen und/oder auf einen digitalen Fingerabdruck reagieren kann. Beispielsweise ist es möglich, bei Überschreitung einer gewissen Herzrate in Kombination mit einer geringen Aktivität eine Warnung auszulösen. Eine solche Warnung kann eine Signalisierung durch das Nutzergerät, eine Benachrichtigung der Auswerteeinrichtung und/oder eines sonstigen Empfangsgerätes sowie eine Speicherung des Ereignisses umfassen.

Erfindungsgemäß findet eine Aggregation, insbesondere im Nutzergerät, statt. Aggregationen liegen vorzugsweise Rohdaten oder ein Rohsignal zu Grunde. Dieses Rohsignal kann von einem, vorzugsweise jedoch mehreren Sensoren stammen. Anhand des konkreten Beispiels eines EKG-Systems können typische EKG-Sensoren kombiniert werden mit Sensorik für die Temperatur, Aktivitätssensoren wie Beschleunigungssensoren und Sensoren für die Umgebungsdaten wie Lage, Licht- oder Akustik-Sensoren. Auch ist eine Kombination mit GPS-Daten aus einem GPS-Modul möglich. Die Rohdaten können aus einer beliebigen Kombination von Daten aus den erwähnten oder sonstigen Quellen bestehen.

Eine erste Möglichkeit der Aggregation liegt in der Veränderung der Auflösungsstufe. So kann eine Datenkompression erfolgen, die vorzugsweise Morphologieerhaltend ist. Dies bedeutet insbesondere, daß eine Datenreduktion vorzugsweise nicht dazu führt, daß relevante Informationen wie beispielsweise Maxima, Peaks oder Unregelmäßigkeiten verloren gehen.

Eine Aggregation erfolgt typischerweise am Ende eines abgeschlossenen Zeitintervalles. Zeitintervalle sind hierarchisch geordnet und mehr oder weniger streng gestuft: Für den menschlichen Betrachter sind Intervallstufen des täglichen Lebens besonders geeignet (z.B. Minuten, Stunden, Tage, Wochen, Monate, Jahre, etc), für eher technische Betrachtungen Intervalle mit logarithmischer Stufung, z.B. 1,10,100,1000,... Sekunden oder 1,3,10,30,100,300,... oder 1,2,5,10,20,50,100,... Vorzugsweise liegen also mehr als eine besonders bevorzugt zwei, drei, vier oder mehr zu Daten korrespondierende Aggregationsstufen im Nutzergerät gleichzeitig vor bzw. sind gleichzeitig abgespeichert.

Neben verschiedenen Möglichkeiten, Aggregations- oder Auflösungsstufen zu wählen, sind unterschiedliche Aggregationsverfahren einsetzbar und/oder kombinierbar. Bezogen auf das Ausführungsbeispiel eines EKG-Systems kann beispielsweise eine Aggregation durch Bestimmung von Parametern wie der Herzrate oder der Normalschlag- Zwischenzeiten erfolgen. Eine Möglichkeit der Aggregation liegt hier darin, insbesondere über unterschiedliche Auslösungsstufen, Analyseverfahren wie solche zur Bestimmung der Herzratenvariabilität zu verwenden. Weiter ist es möglich, eine Aggregation auf Grundlage von Analysen zur Bestimmung von Arrythmien einzusetzen.

Für eine Aggregation können, insbesondere in einem jeweiligen Auflösungsintervall, Ereignisse gezählt, sowie Minima und/oder Maxima gebildet werden. Weiter ist es möglich, die Standardabweichung, den Median, Verteilungskurven oder das nullte, erste oder zweite Moment zu bestimmen. Weitere Möglichkeiten stellen sogenannte Outlier oder Ausreißer dar. Die Aggregation, die Aggregationstiefe und/oder die Auflösungsstufe kann weiter abhängig gemacht werden von weiteren Kriterien wie der Aktivität oder der Lokalisierung eines Patienten.

Für eine Auswertung, insbesondere in oder durch die Auswerteeinrichtung, können die aggregierten Daten in Form einer Signaldarstellung abgebildet werden. Kurven der mittleren Herzraten oder Tachogramme können sowohl klassisch, als auch in Bezug auf die Standardabweichung oder in Bezug auf Häufigkeiten besonderer Ereignisse erfolgen. Eine Auswertbarkeit kann weiter durch eine pixelbasierte- oder graphenbasierte Visualisierung, vorzugsweise durch farbaugmentierte Histogramme erfolgen. Vorzugsweise ermöglicht ein erfindungsgemäßes Verfahren eine interaktive Navigation, durch die, insbesondere aggregierten, Daten. So kann eine zeitliche Visualisierung erfolgen, insbesondere im Hinblick auf die Häufigkeit von Ereignissen und die Verfügbarkeit von Informationen. Vorzugsweise beginnt eine interaktive Navigation mit einer Überblicksdarstellung und einer Selektion von Bereich besonderen Interesses, insbesondere von Zeiträumen. Weiter ist es möglich, Filterkriterien zu definieren, beispielsweise Schwellwerte oder logische Operationen. Alle vorgenannten Signaldarstellungen können für sich oder in beliebiger Kombination miteinander Anwendung finden.

Weiter gibt es unterschiedlichste Möglichkeiten der Darstellung. Dargestellt werden können insbesondere das Tachogramm, zeitadjustierte Graphenüberlagerungen oder Parallelstellungen oder die Häufigkeit von bestimmten Ereignissen. Diese können, insbesondere abhängig von der Aggregationsstufe, visualisiert werden durch X-Y-Graphen, Balken-, Bubble-, Impulsplots, 2D-Graphen, 3D-Graphen und/oder Pixeldarstellungen wie beispielsweise heat-maps.

Als Attribute können beispielsweise Farben, Intensitäten und/oder Sättigungen sowie Formen und Glyphen fungieren.

Für eine interaktive Navigation durch die Daten, die vorzugsweise in aggregierter Form auf dem Nutzergerät gespeichert sind, kann weiter die Drill-Down-Methode verwendet werden. Hierzu werden vorzugsweise interessante Bereiche der, insbesondere aggregierten, Daten identifiziert, markiert und/oder selektiert und in einer anderen, insbesondere verbesserten Auflösungsstufe oder niedrigeren, Aggregationsstufe dargestellt. Die Identifikation findet vorzugsweise durch einen Arzt und/oder mittels der Auswerteeinrichtung statt.

In einem konkreten Beispiel, im Zusammenhang mit einem erfindungsgemäßen EKG-System, werden über einen entsprechenden Sensor elektrische Aktivitäten der Herzmuskelfasern registriert und von einem Nutzergerät als Rohdaten oder Rohsignal gespeichert. Diese Rohdaten werden, insbesondere bereits während der laufenden Aufnahme, aggregiert. Hierzu wird das Rohsignal insbesondere in Minutenabständen mittels Fourieranalyse verarbeitet und ein Wert für die Herzratenvariabilität in den jeweiligen Intervallen bestimmt. Da im Fall der Herzratenvariabilität ein geringer Wert tendenziell als kritisch erachtet wird, kann in einer nächsten Aggregationsstufe eine Minimalwertbildung über beispielsweise 10 Minuten und in folgenden Aggregationsstufen über zunehmende Intervalllängen erfolgen. Auf diese Weise entsteht eine Aggregations-Pyramide unter Verwendung unterschiedlicher Verfahren wie der Fourieranalyse und der Minimalwertbildung sowie der Wahl unterschiedlicher Auflösungsstufen hin zu höheren Aggregationsebenen. Es ist also vorteilhaft, wenn Daten in mehreren Aggregationsstufen gleichzeitig vorliegen, insbesondere im Nutzergerät gespeichert sind. Insbesondere sind die Daten in einer ersten Aggregationsstufe und zusätzlich in mindestens einer zweiten Aggregationsstufe, bevorzugt in drei oder mehr Aggregationsstufen abgespeichert oder werden durch das Nutzergerät erzeugt.

Die Aggregation findet in einer Recheneinheit des Nutzergeräts statt. Weiter sind die Daten von einer externen Auswerteeinrichtung abrufbar. Im konkreten Fall wird beispielsweise eine Mobilfunkverbindung für die Datenübermittlung verwendet, die eine verhältnismäßig geringe Datenübertragungsrate bietet. Die Vorteile der Mobilität des Systems überwiegen insbesondere bei Anwendung des erfindungsgemäßen Verfahrens.

Die Auswerteeinrichtung fordert über die Datenverbindung Messergebnisse von dem Nutzergerät an. Als Antwort auf eine solche Anfrage erhält die Auswerteeinrichtung von dem Nutzergerät jedoch nicht die Rohdaten, sondern bereits aggregierte Daten - im konkreten Fall - beispielsweise die Minimalwerte der Herzratenvariabilität in Bezug auf größere Zeitintervalle. Somit ist nur eine minimale Datenmenge zu übertragen und kann durch die Auswerteeinrichtung beispielsweise als übersichtliches Histogramm dargestellt werden. Erwecken ein oder mehrere Intervalle besonderes Interesse, werden für diese Intervalle zusätzliche, detailliertere Daten angefordert. Sind mehrere zusammenhängende Intervalle von Interesse, wird das Nutzergerät diesen Abschnitt beispielsweise nur um eine Aggregationsstufe detaillierter übermitteln. Wird in einem anderen Fall nur ein sehr kleiner Ausschnitt oder sogar nur ein Intervall gewählt, ist es möglich, eine oder mehrere Aggregationsstufen zu überspringen und gleich eine wesentlich detailliertere Ansicht zu übermitteln. Dieses Prinzip kann soweit fortgesetzt werden, bis für einen, insbesondere besonders interessanten, Bereich oder Ausschnitt, die Rohdaten übermittelt werden. Das Datenaufkommen und die zu der Übertragung notwendige Zeit ist dennoch verhältnismäßig gering, da nur ein kleiner Ausschnitt der Rohdaten übertragen werden muß.

Alternativ zu der Anforderung einer detaillierten Auflösung kann es auch sinnvoll sein, zu der vorliegenden Auflösung oder zu einem Ausschnitt hieraus Daten aus einem anderen Aggregationsverfahren oder einer anderen Aggregationspyramide anzufordern. So ist es erfindungsgemäß auch möglich, nach Identifikation eines interessanten Zeitabschnittes über die Herzratenvariabilität eine, insbesondere ebenfalls aggregierte, Darstellung der Herzrate anzeigen zu lassen. Sollte in Bezug auf die Herzrate die passende Aggregationsstufe noch nicht vorliegen, ist es möglich, daß das Nutzergerät diese auf Anforderung generiert und erst im Anschluß übermittelt oder die Daten einer anderen Aggregationsstufe anbietet. Beispielsweise kann als Aggregation der Herzrate dessen Median bezogen auf ein entsprechendes Zeitintervall dienen. Die übertragenen Werte können durch die Auswerteeinrichtung beispielsweise in Form eines 2D-Graphs visualisiert werden.

Ein anderes Anwendungszenario für das häusliche Umfeld betrifft so genannte "Aktivitäten des täglichen Lebers". Der Anwendung der Erfindung in diesem Bereich kommt im Übrigen eigenerfinderische Bedeutung zu. Anhand von Signalen von Energieverbrauchs-, Präsenz- und anderen Sensoren werden individuelle Verhaltensprofile erkennbar. Diese Informationen sind möglicherweise schützenswert und nur mit Einverständnis der Betroffenen weiterverwendbar. Ein technischer Beschlagnahme- und Missbrauchsschutz kann daher vorgesehen werden. Insbesondere kann das System, das Nutzergerät und/oder die Auswerteeinrichtung, insbesondere zur Vermeidung ungerechtigter oder missbräuchlicher Zugriffe, zur Authentifizierung eines Zugriffs und/oder einer Auswerteeinrichtung und/oder zur Verschlüsselung der Daten ausgebildet sein.

Langzeit-Datenaufzeichnungen sind beispielsweise sachgerecht bzw. vorteilhaft zum einen zum Optimieren von individualisierten Notfalldetektoren, die auf Abweichung von "individuell Typischem" sensibel sind, zum anderen zur Hypothesengenerierung und Befunden von evtl. schleichenden Verhaltensänderungsprozessen, die sich statistisch nachweisen lassen. In beiden oder weiteren Fällen kann die retrospektive Verfügbarkeit von Langzeit-Zeitserien für die Ergebnisqualität entscheidend sein. Eine latente Aufzeichnung und/oder missbrauchssichere, sachgerechte Bereitstellung von Daten im iDataStore ist besonders bevorzugt.

Ein weiteres, auch unabhängig realisierbares Anwendungsbeispiel betrifft einen intelligenten Stromzähler. Ein intelligenter Stromzähler kann als Nutzergerät im Sinne der vorliegenden Erfindung dazu ausgebildet sein, ein oder mehrere Aggregationsverfahren durchzuführen. Beispielsweise kann durch einen Leistungssensor oder dergleichen eine Leistungsaufnahme durch den intelligenten Stromzähler bestimmt und durch eine Rechnereinheit des Stromzählers unter Verwendung eines Aggregationsverfahren verarbeitet und gespeichert werden. Auf diese Weise ist es möglich, einen detaillierten zeitlichen Verlauf der Leistungsaufnahme und/oder aggregierte Daten im Nutzergerät bzw. im intelligenten Stromzähler abzuspeichern. Dies ermöglicht neben den allgemein bekannten Eigenschaften eines intelligenten Stromzählers die Möglichkeit, die eingangs beschriebene Drill-Down-Technik auch hier anzuwenden und, insbesondere schrittweise, detaillierte Daten anzufordern. Somit wird es möglich, beispielsweise mit einem Aggregationsverfahren Fehler wie Fehlerströme oder dergleichen zu detektieren und nachträglich Informationen in einem höheren Detailgrad abzurufen, auch wenn diese sich auf eine Zeit vor dem Fehlerereignis beziehen.

Detaillierte Daten über die Leistungsaufnahme von Verbrauchern an einem korrespondierenden Stromnetz können sensibel sein, da Rückschlüsse auf das Nutzerverhalten möglich sind. Daher kann es bevorzugt sein, dass ein Zugriff auf nichtaggregierte Daten oder Daten mit einem höheren Detailgrad nur vor Ort zur Fehleranalyse möglich ist. Alternativ oder zusätzlich können Verschlüsselungen oder sonstige Verfahren zur Zugriffs-Sicherung vorgesehen sein.

Gemäß der vorliegenden Erfindung können die beschriebenen Aspekte sowohl einzeln als auch in einer beliebigen Kombination realisiert werden. Weitere Aspekte, Vorteile, Merkmale und Eigenschaften der vorliegenden Erfindung ergeben sich aus den Ansprüchen und aus der folgenden Beschreibung eines bevorzugten Ausführungsbeispiels anhand der Zeichnung. Es zeigt
Fig. 1 eine schematische Darstellung eines erfindungsgemäßen Systems und
Fig. 2 eine schematische Darstellung gespeicherter Daten in unterschiedlichen Aggregationsstufen.

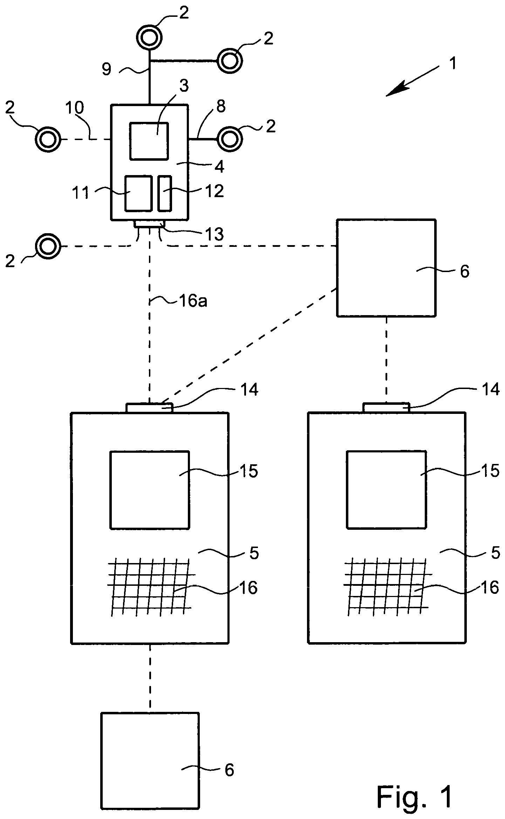

Fig. 1 zeigt ein schematisches Blockschaltbild eines bevorzugten Ausführungsbeispiels eines vorschlagsgemäßen Systems 1 mit Sensoren 2 und einer Rechnereinheit 3, die einem Nutzergerät 4 zugeordnet sind. Weiter weist das System 1 Auswerteeinrichtungen 5 und eine Zentralstelle 6 auf. Sensordaten aus den Sensoren 2 können durch das Nutzergerät 4 abgespeichert werden. Zur Datenübertragung können drahtgebundene Verbindungen, wie Kabel 8 oder Bus-Systeme 9 verwendet werden. Eine weitere Möglichkeit stellt die Anbindung eines Sensors 2 an ein Nutzergerät 4 über eine Funkschnittstelle 10 dar. Das dargestellte Nutzergerät 4 weist weiterhin einen Energiespeicher 11, wie einen Akku oder eine Batterie, eine Ladekontrolleinrichtung 12 sowie eine Schnittstelle 13 auf.

Über die Schnittstelle 13 kann eine Datenverbindung, insbesondere mit der Auswerteeinrichtung 5 oder der Zentralstelle 6, aufgebaut werden. Aufgrund ihrer Flexibilität werden Funkverbindungen bevorzugt. Erfindungsgemäß ist es jedoch auch möglich, daß über die Schnittstelle 13 eine kabelgebundene oder sonstige Datenverbindung hergestellt wird. Bevorzugt wird eine Datenverbindung zwischen der Schnittstelle 13 des Nutzergerätes 4 und der Schnittstelle 14 der Auswerteeinrichtung 5 hergestellt.

Die Auswerteeinrichtung 5 weist bevorzugt eine Anzeigeeinrichtung 15 auf. Zur Steuerung der Auswerteeinrichtung 5 ist weiterhin eine Eingabeeinrichtung 16 vorgesehen. Diese Eingabeeinrichtung 16 ist optional und kann insbesondere durch eine Anzeigeeinrichtung 15 ersetzt werden, die derart ausgestaltet ist, daß sie Funktionen einer Eingabeeinrichtung aufweist. Die Auswerteeinrichtung 5 kann über eine Datenverbindung 16a Daten, insbesondere in einer aggregierten Form, von einem Nutzergerät abrufen und/oder diese von einem Nutzergerät erhalten. Vorzugsweise kann die Auswerteeinrichtung 5 Daten intern abspeichern oder, insbesondere zur Archivierung, zu einer Zentralstelle 6 übermitteln.

Es ist in dem dargestellten Ausführungsbeispiel des Systems 1 vorgesehen, daß das Nutzergerät 4 eine Verbindung zu einer Zentralstelle 6 aufbauen kann. Auf diese Weise kann das Nutzergerät 6 beispielsweise die Informationen über einen kritischen Zustand an die Zentralstelle 6 aktiv übermitteln. Die Zentralstelle 6 ist weiterhin derart ausgestaltet, daß dieses Daten an eine Auswerteeinrichtung 5 übermitteln kann.

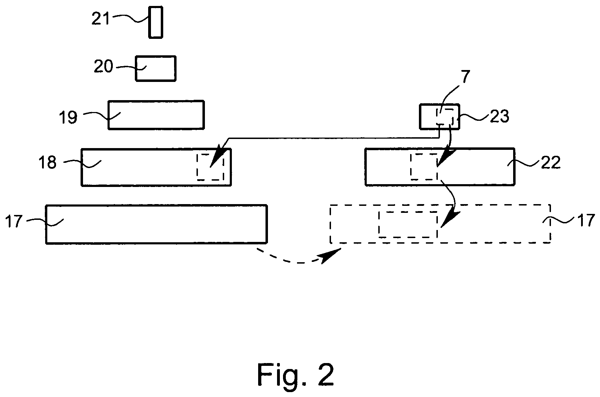

Fig. 2 zeigt in einer schematischen Darstellung eine mögliche Struktur aggregierter Daten. Rohdaten 17 werden im Nutzergerät 4 aggregiert. Ergebnis dieser ersten Aggregation sind die aggregierten Daten 18, die ein verringertes Datenvolumen aufweisen. Weitere Aggregationen führen zu höheren Aggregationsebenen bzw. zu den aggregierten Daten 19, 20 und 21, die ein jeweils abnehmendes Datenvolumen aufweisen. Im Ergebnis entsteht in der abgebildeten Darstellung eine pyramidale Form, wobei die Rohdaten 17 das Fundament bilden mit zunehmender Aggregationsstufe das Datenvolumen in der Darstellung abnimmt, was zu der beschriebenen Aggregations-Pyramide führt.

Unter Verwendung eines anderen Aggregationsverfahrens ist es möglich, aus den Rohdaten 17 von den aggregierten Daten 18 abweichende aggregierte Daten 22 und hieraus wiederum die aggregierten Daten 23 zu erzeugen oder zu berechnen.

An einem Beispiel soll ein Zugriff auf die aggregierten Daten kurz erläutert werden. Eine Teilmenge 7 der aggregierten Daten 23 kann manuell und/oder automatisch und insbesondere unter Verwendung der Auswerteeinheit 5 als interessant identifiziert werden. Vorschlagsgemäß kann diese Teilmenge 7 in einer detaillierteren Aggregationsstufe angefordert werden, indem eine zu der Teilmenge 7 korrespondierende Teilmenge der aggregierten Daten 22 durch die Auswerteeinrichtung 5 angefordert wird.

Alternativ oder zusätzlich ist es möglich, die Teilmenge 7 der Daten aus den Rohdaten 17 oder aus einer anderen Aggregationspyramide auszuwählen. Dies bedeutet insbesondere, daß mit einem anderen Verfahren aggregierte Daten durch die Auswerteeinrichtung 5 abgerufen oder an eine Zentralstelle 6 gesendet werden. Beispielsweise kann eine zu der Teilmenge 7 korrespondierende Teilmenge der aggregierten Daten 18 ausgewählt und übermittelt werden.

### Bezugszeichenliste:

- 1: System
- 2: Sensor
- 3: Rechnereinheit
- 4: Nutzergerät
- 5: Auswerteeinrichtung
- 6: Zentralstelle
- 7: Teilmenge
- 8: Kabel
- 9: Bussystem
- 10: Funkschnittstelle
- 11: Energiespeicher
- 12: Ladekontrolleinrichtung
- 13: Schnittstelle
- 14: Schnittstelle
- 15: Anzeigeeinrichtung
- 16: Eingabeeinrichtung
- 16a: Daten
- 17: Rohdaten
- 18: Daten
- 19: Daten
- 20: Daten
- 21: Daten
- 22: Daten
- 23: Daten

## Patentansprüche

1. Verfahren zur Speicherung und Auswertung von Daten, insbesondere Vitaldaten, wobei die Daten über wenigstens einen Sensor (2) erfasst und von einer Rechnereinheit (3) eines Nutzergerätes (4) gespeichert werden und wobei gespeicherte Daten von einer externen Auswerteeinrichtung (5) abrufbar sind,
wobei die Daten von der Rechnereinheit (3) des Nutzergeräts (4) in hierarchisch aggregierter Form derart gespeichert werden, dass die auf dem Nutzergerät (4) gespeicherten Daten oder Datenteile in aggregierter Form durch die Auswerteeinrichtung (5) über eine digitale Abfrageschnittstelle abrufbar sind,
**dadurch gekennzeichnet,**
**dass** die Daten von der Rechnereinheit (3) des Nutzergeräts (4) in hierarchisch aggregierter Form für eine hierarchische Datennavigation so präpariert werden, dass sie in diversen zeitlich und/oder semantisch komprimierten Formen ausgelesen werden können, wobei auf einen Satz Rohdaten unterschiedliche Aggregationsverfahren alternativ oder kumulativ angewendet werden, wobei das Datenvolumen mit zunehmender Aggregation sinkt.

2. Verfahren nach Anspruch 1, **dadurch gekennzeichnet, dass** die Daten in einer ersten Aggregationsstufe und zusätzlich in mindestens einer zweiten Aggregationsstufe abgespeichert werden und/oder durch die Auswerteeinrichtung (5) abrufbar sind.

3. Verfahren nach einem der Ansprüche 1 oder 2, **dadurch gekennzeichnet, dass** eine Teilmenge der Daten in einer aggregierten Form, insbesondere unter Verwendung der Auswerteeinrichtung (5), manuell und/oder automatisch als interessant identifiziert werden und diese Teilmenge der Daten in einer detaillierteren Aggregationsstufe und/oder in einer anderen aggregierten Form durch die Auswerteeinrichtung (5) angefordert und/oder durch das Nutzergerät übermittelt werden.

4. Verfahren nach einem der voranstehenden Ansprüche, **dadurch gekennzeichnet, dass** die Daten durch das Nutzergerät (4) in einem netzunabhängigen Betrieb erfasst werden.

5. Verfahren nach einem der voranstehenden Ansprüche, **dadurch gekennzeichnet, dass** die Daten drahtlos durch die Auswerteeinrichtung (5) abgerufen oder drahtlos an diese übermittelt werden, insbesondere in ihrer aggregierten Form.

6. Verfahren nach einem der voranstehenden Ansprüche, **dadurch gekennzeichnet, dass** die Rechnereinheit (3) im Nutzergerät (4) zur Aggregation die über wenigstens einen Sensor (2) erfassten Daten in, insbesondere einem zeitlichen Intervall zugeordnete, Datenteile unterteilt, und dass, vorzugsweise, auf diese Datenteile jeweils ein Aggregationsverfahren angewendet wird.

7. Verfahren nach einem der voranstehenden Ansprüche, **dadurch gekennzeichnet, dass** Daten mittels Unterabtastung, statistischer Analyse, Berechung abgeleiteter Signalbeschreibungen, nichtlinearer Transformationen, insbesondere FFT, DCT, Wavelet-Transformation, jeweils insbesondere mit komprimierender Ausdünnung, und/oder mittels Verfahren des maschinellen Lernens aggregiert werden.

8. Verfahren nach einem der voranstehenden Ansprüche, **dadurch gekennzeichnet, dass** die Auswahl eines Aggregationsverfahrens und/oder die Anzahl durchzuführender Aggregationsvorgänge durch die Rechnereinheit (3) in Abhängigkeit von einem Ladezustand des Nutzergeräts (4) erfolgt.

9. Verfahren nach einem der voranstehenden Ansprüche, **dadurch gekennzeichnet, dass** maschinelle Lernverfahren auf Basis historischer Daten und/oder interative explorative Dataminingverfahren verwendet werden.

10. System (1) zur Auswertung von Daten, insbesondere Vitaldaten, ausgebildet zur Durchführung des Verfahrens nach einem der voranstehenden Ansprüche.

11. System nach Anspruch 10, **dadurch gekennzeichnet, dass** wenigstens ein mit einer externen Auswerteeinrichtung (5) kommunizierendes Nutzergerät (4) vorgesehen ist, dass das Nutzergerät (4) wenigstens einen zugeordneten Sensor (2) zur Erfassung von Daten und eine Rechnereinheit (3) zur Speicherung der Daten aufweist und dass die Rechnereinheit (3) derart ausgebildet ist, dass die Daten von der Rechnereinheit (3) des Nutzergeräts (4) in hierarchisch aggregierter Form auf dem Nutzergerät (4) derart gespeichert werden, dass die auf dem Nutzergerät (4) gespeicherten Daten oder Datenteile in aggregierter Form durch die Auswerteeinrichtung (5) abrufbar sind.

12. System nach einem der Ansprüche 10 oder 1 1, **dadurch gekennzeichnet, dass** System (1), das Nutzergerät (4) und/oder die Auswerteeinrichtung (5), insbesondere zur Vermeidung unberechtigter oder missbräuchlicher Zugriffe, zur Authentifizierung einer Auswerteeinrichtung (5) und/oder zur Verschlüsselung der Daten ausgebildet ist.

13. Nutzergerät (4) für ein System (1) nach einem der Ansprüche 10 bis 12, **dadurch gekennzeichnet, dass** wenigstens ein zugeordneter Sensor (2) zur Erfassung von Daten und eine Rechnereinheit (3) zur Speicherung der Daten vorgesehen ist und dass die Rechnereinheit (3) des Nutzergeräts (4) derart ausgebildet ist, dass die Daten von der Rechnereinheit (3) in hierarchisch aggregierter Form auf dem Nutzergerät (4) so gespeichert werden, dass die Daten in aggregierter Form durch die Auswerteeinrichtung (5) abrufbar sind,
**dadurch gekennzeichnet,**
**dass** die Daten für eine hierarchische Datennavigation so präpariert werden, dass sie in diversen zeitlich und/oder semantisch komprimierten Formen ausgelesen werden können, wobei auf einen Satz Rohdaten unterschiedliche Aggregationsverfahren alternativ oder kumulativ angewendet werden,
wobei das Datenvolumen mit zunehmender Aggregation sinkt, wodurch es möglich ist, sich die mehrstufig aggregierten Daten als Pyramide vorzustellen, bei der die Rohdaten mit dem größten Datenvolumen das Fundament der Pyramide darstellen und hierauf aufbauend mit jeder Aggregationsstufe oder -ebene ein verringertes Datenvolumen einhergeht.

14. Nutzergerät (4) nach Anspruch 13, **dadurch gekennzeichnet, dass** das Nutzergerät (4), insbesondere durch einen Akkumulator oder eine Batterie, netzunabhängig oder mobil betreibbar ist.

15. Auswerteeinrichtung (5) für ein System (1) nach einem der Ansprüche 10 bis 12, **dadurch gekennzeichnet, dass** auf dem Nutzergerät (4) gespeicherte Daten oder Datenteile, die für eine hierarchische Datennavigation so präpariert sind, dass sie in diversen zeitlich und/oder semantisch komprimierten Formen ausgelesen werden können, wobei auf einen Satz Rohdaten unterschiedliche Aggregationsverfahren alternativ oder kumulativ angewendet werden, wobei das Datenvolumen mit zunehmender Aggregation sinkt, wodurch es möglich ist, sich die mehrstufig aggregierten Daten als Pyramide vorzustellen, bei der die Rohdaten mit dem größten Datenvolumen das Fundament der Pyramide darstellen und hierauf aufbauend mit jeder Aggregationsstufe oder -ebene ein verringertes Datenvolumen einhergeht, durch die Auswerteeinrichtung (5) selektierbar, abrufbar und/oder anzeigbar sind.

## Claims

1. Method for saving and analysing data, particularly vital data, wherein the data is detected by at least one sensor (2) and saved by a processing unit (3) of a user terminal (4) and wherein saved data can be retrievable by an external evaluating device (5),
wherein the data is saved by the processing unit (3) of the user terminal (4) in a hierarchically aggregated format such that the data or data parts saved on the user terminal (4) are retrievable in aggregated format by the evaluating device (5) via a digital query interface,
**characterised in that**
the data is prepared by the processing unit (3) of the user terminal (4) in a hierarchically aggregated format for hierarchical data navigation such that it can be read in various chronologically and/or semantically condensed forms, wherein various aggregation methods can be applied alternatively or cumulatively to one set of raw data, wherein the data volume declines with increasing aggregation.

2. Method according to claim 1, **characterised in that** the data is saved in a first aggregation level and additionally in at least a second aggregation level and/or can be retrieved by the evaluating device (5).

3. Method according to one of claims 1 or 2, **characterised in that** a subset of the data in an aggregated format is identified manually and/or automatically as interesting, particularly by using the evaluating device (5), and this subset of data is requested in a more detailed aggregation level and/or in another aggregated format by the evaluating device (5) and/or transmitted by the user terminal.

4. Method according to one of the preceding claims, **characterised in that** the data is detected by a user terminal (4) in a network-independent operation.

5. Method according to one of the preceding claims, **characterised in that** the data is requested wirelessly by the evaluating device (5) or transmitted wirelessly to this, particularly in its aggregated form.

6. Method according to one of the preceding claims, **characterised in that** the processing unit (3) in the user terminal (4) for aggregation divides the data being detected by at least one sensor (2) into data parts assigned particularly to a time interval and that, preferably, an aggregation method is applied respectively to these data parts.

7. Method according to one of the preceding claims **characterised in that** data is aggregated by means of subsampling, statistical analysis, calculation of derived signal descriptions, non-linear transformations, particularly FFT, DCT, wavelet transformation, respectively particularly with compressing thinning, and/or by means of the method of machine learning.

8. Method according to one of the preceding claims, **characterised in that** the selection of an aggregation method and/or the quantity of aggregation operations to be performed occurs by the processing unit (3) depending on a charge state of the user terminal (4).

9. Method according to one of the preceding claims, **characterised in that** machine learning methods are used on the basis of historical data and/or interactive, explorative data mining methods.

10. System (1) for evaluating data, particularly vital data, designed to execute the operation according to one of the preceding claims.

11. System according to claim 10, **characterised in that** at least one user terminal (4) communicating with an external evaluating device (5) is provided, **in that** the user terminal (4) has at least one assigned sensor (2) for detecting data and one processing unit (3) for saving the data and **in that** the processing unit (3) is designed such that the data is saved by the processing unit (3) of the user terminal (4) in a hierarchically aggregated format on the user terminal (4) such that the data or data parts saved on the user terminal (4) are retrievable in aggregated format by the evaluating device (5).

12. System according to one of claims 10 or 11, **characterised in that** the system (1), the user terminal (4) and/or the evaluating device (5) is designed particularly for avoiding unauthorised or improper access, for authenticating an evaluating device (5) and/or for encoding the data.

13. User terminal (4) for a system (1) according to one of claims 10 to 12, **characterised in that** at least one assigned sensor (2) for detecting data and one processing unit (3) for saving the data is provided and **in that** the processing unit (3) of the user terminal (4) is designed such that the data is saved by the processing unit (3) in a hierarchically aggregated format on the user terminal (4) such that the data is retrievable in aggregated format by the evaluating device (5),
**characterised in that**
the data is prepared for hierarchical data navigation such that it can be read in various chronologically and/or semantically condensed forms, wherein various aggregation methods can be applied alternatively or cumulatively to one set of raw data,
wherein the data volumes decline with increasing aggregation, whereby it is possible to present the multi-level aggregated data as a pyramid, wherein the raw data with the greatest data volume represents the foundation of the pyramid and building thereon a reduced data volume accompanies each aggregation level or plane.

14. User terminal (4) according to claim 13, **characterised in that** the user terminal (4) can be operated network-independently or mobile, particularly by means of an accumulator or battery.

15. Evaluating device (5) for a system (1) according to one of claims 10 to 12, **characterised in that** data or data parts being saved on the user terminal (4), which are prepared for hierarchical data navigation such that they can be read in various chronologically and/or semantically compressed formats, wherein various aggregation methods can be applied alternatively or cumulatively to one set of raw data, wherein the data volumes decline with increasing aggregation, whereby it is possible to present the multi-level aggregated data as a pyramid, wherein the raw data with the greatest data volume represents the foundation of the pyramid and building thereon a reduced data volume accompanies each aggregation level or plane, are selectable, retrievable and/or displayable by the evaluating device (5).

## Revendications

1. Procédé de mise en mémoire et d'évaluation de données, en particulier de données vitales, les données étant détectées par au moins un capteur (2) et mises en mémoire par une unité informatique (3) d'un équipement utilisateur (4) et les données mises en mémoire pouvant être appelées par un dispositif d'évaluation externe (5),
les données étant mises en mémoire par l'unité informatique (3) de l'équipement utilisateur (4) sous forme hiérarchiquement agrégée de sorte que les données ou les divisions de données mises en mémoire dans l'équipement utilisateur (4) ou les parties de données sous forme agrégée puissent être appelées par le dispositif d'évaluation (5) au moyen d'une interface d'interrogation digitale,
**caractérisé en**
**ce que** les données sont préparées par l'unité informatique (3) de l'équipement utilisateur (4) sous forme hiérarchiquement agrégée pour une navigation de données hiérarchique, de sorte qu'elles puissent être lues sous diverses formes comprimées sémantiquement et/ou temporellement, différents procédés d'agrégation pouvant être utilisés alternativement ou cumulativement sur un ensemble de données brutes, le volume de données se réduisant à mesure que l'agrégation augmente.

2. Procédé selon la revendication 1, **caractérisé en ce que** les données sont mises en mémoire dans une première étape d'agrégation et en plus dans au moins une seconde étape d'agrégation et/ou sont appelées par le dispositif d'évaluation (5).

3. Procédé selon l'une quelconque des revendications 1 ou 2, **caractérisé en ce qu'**une quantité partielle des données sous une forme agrégée, en particulier au moyen du dispositif d'évaluation (5) est identifiée manuellement et/ou automatiquement comme intéressantes et ladite quantité partielle des données dans une étape d'agrégation plus détaillée et/ou sous une autre forme agrégée est demandée par le dispositif d'évaluation (5) et/ou est transmise par l'équipement utilisateur.

4. Procédé selon l'une quelconque des revendications précédentes, **caractérisé en ce que** les données sont collectées par l'équipement utilisateur (4) dans un fonctionnement autonome.

5. Procédé selon l'une quelconque des revendications précédentes, **caractérisé en ce que** les données sont appelées sans fil par le dispositif d'évaluation (5) ou lui sont transmises sans fil, en particulier sous leur forme agrégée.

6. Procédé selon l'une quelconque des revendications précédentes, **caractérisé en ce que** l'unité informatique (3), dans l'équipement utilisateur (4) pour l'agrégation, divise les données détectées par au moins un capteur (2) en divisions de données associées en particulier à un intervalle temporel, et **en ce que** de préférence, un procédé d'agrégation respectif est utilisé sur lesdites divisions de données.

7. Procédé selon l'une quelconque des revendications précédentes, **caractérisé en ce que** les données sont agrégées au moyen de sous-balayage, d'analyse statistique, de calcul de descriptions de signal dérivées, transformations non linéaires, en particulier FFT, DCT, transformée en ondelettes, respectivement en particulier par amincissement de compression, et/ou au moyen de procédé de l'apprentissage machine.

8. Procédé selon l'une quelconque des revendications précédentes, **caractérisé en ce que** la sélection d'un procédé d'agrégation et/ou le nombre de processus d'agrégation à effectuer est effectuée par l'unité informatique (3) en fonction d'un état de charge de l'équipement utilisateur (4).

9. Procédé selon l'une quelconque des revendications précédentes, **caractérisé en ce que** les procédés d'apprentissage machine sont utilisés sur la base de données historiques et/ou de procédé d'exploration de données exploratoire interactive.

10. Système (1) d'évaluation de données, en particulier de données vitales, conçu pour exécuter le procédé selon l'une quelconque des revendications précédentes.

11. Système selon la revendication 10, **caractérisé en ce qu'**il est prévu au moins un équipement utilisateur (4) en communication avec un dispositif d'évaluation externe (5), **en ce que** l'équipement utilisateur (4) comporte au moins un capteur associé (2) pour détecter des données et une unité informatique (3) pour enregistrer les données et **en ce que** l'unité informatique (3) est conçue de telle sorte que les données soient enregistrées par l'unité informatique (3) de l'équipement utilisateur (4) sous forme agrégée hiérarchiquement dans l'équipement utilisateur (4) de telle sorte que les données ou les divisions de données enregistrées dans l'équipement utilisateur (4) puissent être appelées sous forme agrégée par le dispositif d'évaluation (5).

12. Système selon l'une quelconque des revendications 10 ou 11, **caractérisé en ce que** le système (1), l'équipement utilisateur (4) et/ou le dispositif d'évaluation (5), est conçu en particulier pour empêcher des accès non autorisés ou abusifs, pour authentifier un dispositif d'évaluation (5) et/ou pour chiffrer des données.

13. Équipement utilisateur (4) pour un système (1) selon l'une quelconque des revendications 10 à 12, **caractérisé en ce qu'**on prévoit au moins un capteur associé (2) destiné à la détection de données et une unité informatique (3) destinée à l'enregistrement de données et **en ce que** l'unité informatique (3) de l'équipement utilisateur (4) est conçu de telle sorte à ce que les données soient enregistrées par l'unité informatique (3) sous forme agrégée hiérarchiquement dans l'équipement utilisateur (4) de telle sorte que les données sous forme agrégée puissent être appelées par le dispositif d'évaluation (5),
**caractérisé en**
**ce que** les données sont préparées pour une navigation de données hiérarchique de manière à ce qu'elles puissent être lues sous diverses formes comprimées temporellement et/ou sémantiquement, différents procédés d'agrégation étant utilisés en alternance ou ensemble sur un ensemble de données brutes,
selon lequel le volume de données se réduisant à mesure que l'agrégation augmente, ainsi il est possible de présenter les données agrégées en plusieurs étages comme une pyramide, dans laquelle les données brutes ayant le plus gros volume de données représentent la fondation de la pyramide et ensuite un volume de données réduit est associé à chaque étape ou plan d'agrégation.

14. Équipement utilisateur (4) selon la revendication 13, **caractérisé en ce que** l'équipement utilisateur (4) peut être utilisé de manière autonome et mobile, en particulier au moyen de batterie ou accumulateur.

15. Dispositif d'évaluation (5) pour un système (1) selon l'une quelconque des revendications 10 à 12, **caractérisé en ce que** les données ou des divisions de données enregistrées dans l'équipement utilisateur (4), qui sont préparées pour une navigation de données hiérarchique de telle sorte qu'elles puissent être lues sous diverses formes comprimées temporellement et/ou sémantiquement, différents procédés d'agrégation étant utilisés en alternance ou ensemble sur un ensemble de données brutes, le volume de données se réduisant à mesure que l'agrégation augmente, permettant ainsi de présenter les données agrégées en plusieurs étages comme une pyramide, dans laquelle les données brutes ayant le plus gros volume de données représentent la fondation de la pyramide et ensuite un volume de données réduit est associé à chaque étape ou plan d'agrégation, peuvent être sélectivement appelées et/ou affichées par le dispositif d'évaluation (5).
